(19) **Europäisches Patentamt**

**European Patent Office**

**Office européen des brevets**

(11) **EP 1 313 531 B1**

(12) **EUROPEAN PATENT SPECIFICATION**

(45) Date of publication and mention
of the grant of the patent:
**13.07.2005 Bulletin 2005/28**

(21) Application number: **01967860.6**

(22) Date of filing: **24.08.2001**

(51) Int Cl.⁷: **A61N 7/00**

(86) International application number:
**PCT/NO2001/000349**

(87) International publication number:
**WO 2002/015976 (28.02.2002 Gazette 2002/09)**

(54) **APPARATUS FOR SELECTIVE CELL DESTRUCTION WITHIN A LIVING ORGANISM**

GERÄT ZUR SELEKTIVEN ZELLZERSTÖRUNG IN EINEM LEBENDEM ORGANISMUS

APPAREIL DE DESTRUCTION SELECTIVE DE CELLULES DANS UN ORGANISME VIVANT

(84) Designated Contracting States:
**AT BE CH CY DE DK ES FI FR GB GR IE IT LI LU
MC NL PT SE TR**

(30) Priority: **25.08.2000 NO 20004286**

(43) Date of publication of application:
**28.05.2003 Bulletin 2003/22**

(73) Proprietor: **Cancer Cure AS
1358 Jar (NO)**

(72) Inventor: **Myhr, Gunnar
N-1358 Jar (NO)**

(74) Representative: **Grünecker, Kinkeldey,
Stockmair & Schwanhäusser Anwaltssozietät
Maximilianstrasse 58
80538 München (DE)**

(56) References cited:
**WO-A1-00/24468         WO-A1-83/02718
WO-A1-98/55605          DE-A1- 4 414 239
US-A- 5 209 234**

## Description

## 1. Introduction

The invention is concerning an apparatus for selective cell destruction within a living organism like a human being, animal or a plant, subjected to an acoustic transmitter arranged to transmit an acoustic signal into a first tissue portion within the organism, and a guidance/control system arranged to control characteristics associated with the acoustic signal, including the determination of the frequency of at least one signal component of the acoustic signal to a specific frequency value in such a way that certain cells within the first tissue portion is damaged or destroyed under the influence or exposure of the signal.

A methodology related to the introductory mentioned apparatus is previously known from US patent no. 4,315,514. The technique which is described in this publication has severe disadvantages, which are discussed later in this description. An apparatus according to the preamble of claim 1 is disclosed in US-A-4,315,514.

An additional device of the introductory mentioned type is known from German patent no. 44 14 239. This publication is concerning selective cell destruction based on an acoustic signal with a certain frequency equivalent to the resonance frequency of the cell, which again may destroy or damage the cell. The technique described in this publication have the primary drawbacks;

- It requires a simulation system to determine the right resonance frequency

- It does not include guidance and control with respect to energy intensity or total energy supplied

- It does not include other mechanisms to cause selective cell destruction

Other disadvantages are described later in this document.

An object of the present invention is to provide an apparatus which does not have these disadvantages and at the same time includes several modes of selective cell destruction.

In accordance with the invention the above object is achieved by the apparatus defined in the independent claim 1. Additional favourable features are provided by the dependent claim.

In the figures,

Figure 1 illustrates normal and cancer cells from the surface of the human uterine cervix. Picture a shows normal cells, picture b shows cells in dysplasia stage, picture c represents invasive carcinoma, Alberts (1995).

Figure 2 shows typical abnormalities in the appearance of the nucleus of a cancer cell (erythroleukaemia cell), Alberts (1995).

Figure 3 is a schematic presentation of a model of a cell system.

Figure 4 is a graph illustrating amplitude rate as a function of $\omega/\omega_n$ for various h/k ratios.

Figure 5 illustrates an experimental set up of mouse foot, T-arrangement, fixtures and beaker with degassed water.

Figure 6 illustrates an overall experimental set up of hardware and software (B&K = Brüel & Kjær).

Figure 7 illustrates an overview of mouse leg with tumour.

Figure 8 is a graph illustrating absorption rates (in Pascal) as a function of frequency for a healthy back leg, Balb/c nude mouse.

Figure 9 is a graph illustrating absorption rates (in Pascal) as a function of frequency for a back leg with tumour, Balb/c nude mouse.

Figure 10 is a schematic block diagram of an apparatus for selective cell or virus destruction.

Figure 11 is a block diagram for an apparatus for selective cell or virus destruction.

Figure 12 is a block diagram for scanline processing of reflected signal, as a function of depth, for measurement and monitoring of tissue.

Figure 13 is a scanline processing unit for PW ("Pulse Wave")/CW ("Continuous Wave") doppler.

Figure 14 is a block diagram for a scanline processing unit for colour and flow imaging.

## 2. Background - cell biology

The cell is one of the most basic elements concerning life. There are millions of different types of cells, from the simplest one-celled organism to cells that only function when they are part of an organ within a larger complex organism.

All cells have an external plasma membrane, protecting them from the outside environment. The cell membrane regulates the movement of water, nutrients and waste into and out of the cell. Cells are divided into two categories; those that have a nucleus, eukaryotic cells, and those that do not have a membrane-enclosed nucleus, prokaryotic cells. Information is stored in chromosomes. In eukaryotic cells the chromosomes are located in the nucleus.

The internal structure of a eukaryotic cell comes from the cytoskeleton, a network of protein filaments and microtubules. The cytoskeleton is involved in cell movement, cell shape and movement of materials within the cell. Within the cytosol there are several membrane enclosed organelles. Animal, plant, protist and fungal cells contain mitochondria which are membrane-bound organelles that use oxygen to generate energy from food. Mitochondria contain their own DNA. In addition to mitochondria, plants contain chloroplasts, which are membrane-bounded organelles that carry out photosynthesis. Like mitochondria, chloroplasts contain their own DNA. Eukaryotic cells also contain other membrane-bounded spaces including the endoplasmic reticulum, the Golgi apparatus, lysosomes and peroxisomes.

All cells contain ribosomes which are the sites of protein synthesis. Ribosomes are found within the cytosol. Although eukaryotic and prokaryotic ribosomes are similar in structure and function, there is a definitive difference between these ribosomes which reflects the fundamental difference between the two basic cell types.

Prokaryotic cells are much smaller and simpler than eukaryotic cells. Typically they are only 1 to several microns ($10^{-6}$ meters) in diameter, whereas a typical eukaryotic cell is about 20 microns in diameter. In addition to lacking a aiembrane-confined nucleus, prokaryotes have no mitochondria or chloroplasts.

For further discussions one may refer to e.g. Alberts (1995) and NIH (2001).

A cancer tumour has its origin from the genetic mutation of some cells within a cell population which increases the propensity for further cell division (growth).

The mutated cell has an external geometry which appear normal, but it can continue to reproduce. This stage is labelled hyperplasia. After several years a small proportion of these cells may proceed with additional mutations with increased loss of control with respect to growth. At this stage the cell's shape and orientation start to divagate. After the mutation of approximately 10 of the original cell's growth controlling genes, the cell has obtained additional growth and non-normal configuration. At this stage there is an in situ cancer if the tumour has not penetrated surrounding tissue. A tumour at this stage can in principle be encysted indefinitely. After additional mutations the tumour can penetrate surrounding tissue and shed cancer cells to the blood or lymph system, which may cause metastases and the development of tumours in other body parts.

For further discussions one may refer to e.g. Fleming (1997) and NCI (2001).

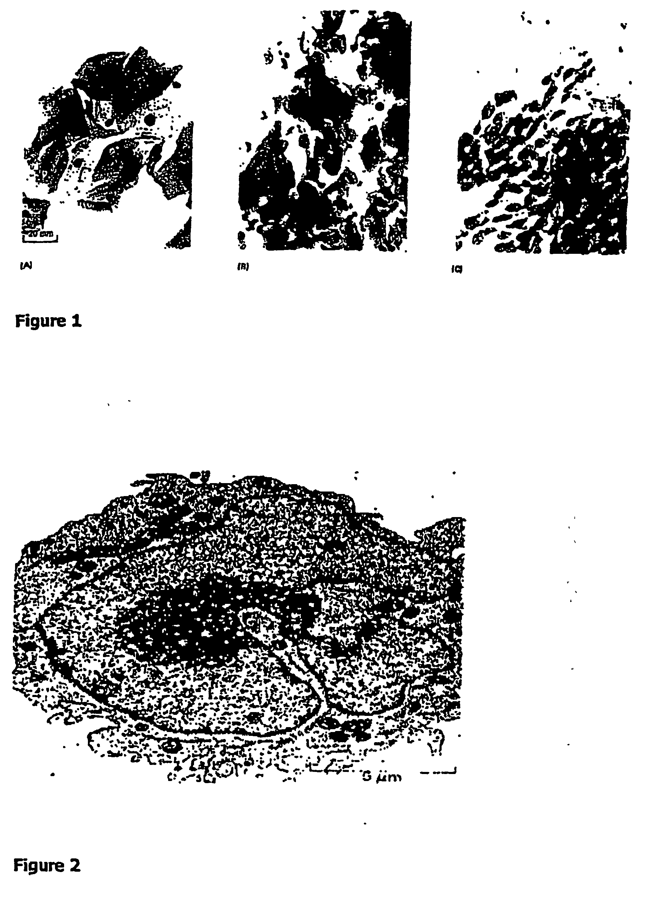

Figure 1 shows both normal and cancer cells from the surface of the uterine cervix. Picture a shows normal cells, which are large and well differentiated, with highly condensed nuclei. Picture b shows cells in dysplasia stage. The cells are in a variety of stages of differentiation. Picture c represents invasive carcinoma, the cells all appear undifferentiated, with scanty cytoplasm and a relatively large nucleus. Picture 2 shows typical abnormalities in the appearance of the nucleus of a cancer cell, in this example an erythroleukemia cell. The cancer cell's nucleus is large in relation to the amount of cytoplasm, with an irregularly intented envelope and a nucleus that is also abnormally large and complex in its structure.

More than 200 types of cancer have been categorised, all which are characterised by uncontrolled growth and metastasis.

Cancer cells distinguish themselves from normal cells by the following external and internal physical structure and characteristics:

- Increased metabolic rates and transport of substances across the plasma membrane

- Loss of cytoskeletal structures

- Reduced attachment to other cells

- Rounded shape

- Alterations in structure and density of surface carbohydrate groups

- Partial or complete loss of differentiation

- High nucleus-to-cytoplasm ratio

- Abnormally large nucleus with a complex structure

The higher rates of metabolism in cancer cells have resulted in higher resting temperatures compared to normal cells. The optimal (average) temperature for a cancer cell is known to be 37.5 °C while that for a normal cell is 37°C. Another physical characteristic that differentiates the cancer cells from the normal cells, is that cancer cells die at lower temperatures than do normal cells. The temperature at which a normal cell exposed to hyperthermia will be killed and thereby irreversibly will be unable to perform normal cell functions is about 46.5 °C, on the average. The cancer cell, in contrast, will be killed at about 45.5 °C. The temperature elevation increment necessary to cause death in the cancer cell is determined to be at least approximately 8.0 °C, while the normal cell can withstand a temperature increase of at least 9.5 °C (US patent no. 4,622,952).

**2.1 Background - oncology**

Traditional treatment with respect to cure or alleviation from cancer have been combinations of the following approaches;

- Surgery

- Radiation treatment

- Chemotherapy

- Hormone treatment

- Critical observation

For an in depth discussion of these treatment options one may refer to e.g. NCI (2001).
Of the future treatment options that are emerging, one may mention;

- Hyper-thermal treatment, to increase the tissue temperature locally to destroy cancer cells and/or make traditional radiation treatment more effective (ref. paragraph 5.1)

- Gene therapy, related topics include; antisense technology, drug resistance, hematopoietic gene transfer, homologous recombination, ribosome technology, tumour immunotherapy, tumour suppressors

- Immune therapy, to stimulate the body's own immune system

- Molecular therapy, on molecular level repair damaged DNA and/or obtain the blockage of certain growth proteins, and/or enhancing the sensitivity of tumours with respect to conventional treatment

- Photodynamic therapy, the combination of chemicals and light

- Anti-angiogenic drugs, the interference with new capillaries to cancer tumours by angiogenesis inhibitors

For extensive discussions of experimental/new treatment options one may refer to JNCI (2001) and NCI (2001).

**3. Objective of the invention and bounds to related technologies**

Traditional treatment of cancer have been combinations of medicine (surgery) and biochemical processes.

The major problem has been to differentiate between cancer cells and normal cells, that cancer cells have developed resistance against chemotherapy, in combination with metastases and/or critical location of tumours. Any sufficient answers to these challenges will probably not be provided by the prospective new treatment options mentioned under paragraph 2.1.

An approach that has previously not been used in the treatment of cancer, is to utilise the differences in both internal and external physical structure and/or properties, and as such, to use these differences to differentiate between cancer cells and normal cells, and at the same time utilise the differences in physical properties to selectively attack and destroy cancer cells specifically by external mechanical stress and strain, isolated or in combination with (internally) induced apoptosis and/or necrosis or in additional combination with traditional methods of treatment like chemotherapy, in combination with (ultra)sonic shock.

A patent that may relate to one of the treatment modes applied in this document, is the methodology for the application of resonance frequencies in US patent no. 4,315,514. Based on a weak theoretical foundation, the claims are defined for a methodological application of resonance frequencies and determination of a damping factor to destroy certain (cancer) cells without damaging other (healthy) cells. The methodology demands a certain transmission path and a biopsy to determine resonance frequencies and damping.

The approach by the provided invention, represented by the stated apparatus, algorithm, procedure and treatment options distinguishes itself from US patent no. 4,315,514, among others, by;

- Represents a well defined algorithm to guidance and control of a well defined apparatus for selective cell and virus destruction

- The invention constitutes a well defined and structured system with transmitter(s)/receiver(s), scanline processors, central processing unit, other processors, visualisation, etc

- The apparatus is not dependent on a specific transmission path

- The apparatus can be utilised in a diagnostic contexts (both medically and for frequency determination) and as such it is not dependent on a biopsy

- The apparatus, from the scanline processor and/or from general energy absorption measurements, provides feedback in true time, by endogenous measurements of intensities, accumulated energy and the control of transmitter (s) with respect to intensities and transmission time according to empirical data

- The various modes of effect which the algorithm controls, may combine the various modes in parallel and/or sequentially; external mechanical effects with internally induced apoptosis/necrosis, and in addition to the combination with traditional treatments in general and chemotherapy with acoustic shock in particular.

Most of the above stated objections are also valid for German patent no. 44 14 239. In addition, the described system in this patent requires the use of a finite element simulation system for the determination of natural frequencies.

An approach which conceptually might touch the starting point of the stated approach, is represented by US patent no. 4,622,952. In this method one is utilising the differences in magnetic resonance absorption frequencies. This is a process for the treatment of cancer by the application of external electromagnetic energy capable of achieving biophysical alterations in the intracellular structure of cancer cells in living tissue, including stimulation of intracellular production of interferon. The process accomplishes these biophysical alterations by tuning an external electromagnetic energy to the resonant energy absorption frequencies of the intracellular structure of the selected cells and then exposing the subject to this tuned electromagnetic energy field. Alternatively, the field can be tuned to the frequency which has been calculated to be closest to the resonant frequency of the cancer cells and furthest from the resonant frequency of the normal cells. The process may be further enhanced by the intracellular absorption of selected materials designed to alter the magnetic susceptibility and therefore the resonant energy absorption frequency of the intracellular structure.

US patent no. 5,899,857 may also be appropriate to mention in the sense it is close to US patent no. 4,622,952, but in this case one is utilising monochromatic electromagnetic energy for the destruction of organic material.

The purpose of the invention in this document is to define an apparatus and algorithm for selective cell destruction which e.g. endogenously measures intensities and accumulated energy along scanlines and which controls transmitters with respect to intensities and time according to empirical data.

This may represent an independent alternative and/or being a supplement to traditional and/or future treatment options which are described under paragraph 2.1, in addition to represent an independent and complete treatment option for other bacteria diseases or virus infections.

It is an integral part of the overall concept, related to the application of the provided invention that it may provide the basis for the treatment of a larger body part or whole organ without the possibility of damaging healthy tissue.

The three modes of selective cell destruction for the treatment of cancer, all which are acoustically related, are included within the stated apparatus, algorithm and procedure. These modes of attacking cancer cells are discussed in the text to follow. Firstly, a discussion of selective apoptosis/necrosis is provided, followed by an analysis of the application of acoustic shock in combination with conventional treatments (chemotherapy). Thirdly, an analysis of cell destruction by the application of natural frequency is treated. Some empirical evidence related to absorption frequency is also provided.

## 4. Modes of selective cell destruction

### 4.1 By inducing apoptosis/necrosis

Apoptosis is a mechanism by which cells are programmed to die under a wide range of physiological, biochemical and developmental stimuli. From the perspective of cancer, apoptosis is both a mechanism which suppresses tumour genesis and is a predominant pathway in antineoplastic therapy. Many cancer cells circumvent the normal apoptotic mechanisms to prevent their self-destruction because of the many mutations they harbour. Thus, disarming apoptosis and other surveillance mechanisms is of fundamental significance in allowing the development of the malignant and metastatic phenotype of a cancer cell.

Apoptosis is a distinct morphological form of cell death which can be characterised by cell shrinkage, membrane disruption and chromatin condensation which finally leads to cell fragmentation. A striking characteristic of apoptosis is the formation of hyperchromatic nuclei containing DNA that is then redistributed to the nuclear margins. For a general treatment of apoptosis, see e.g. Kumar (1999).

Therapeutic ultrasound (ULS) and the resulting cavitational process has been shown to induce irreversible cell damage. In a study, Ashush (2000), high intensity focused pulsed ULS sonication at a frequency of 750 kHz was delivered to HL-60, K562, U937, and M1/2 leukaemia cell line cultures. ULS exposure was used with induction of transient cavitation in the focal area, delivered with an intensity level of 103.7 $W/cm^2$ and 54.6 $W/cm^2$, spatial-peak temporal-average intensity. As a control, ULS of lower intensity was delivered at 22.4 $W/cm^2$, spatial-peak temporal-average intensity. In this study the following morphological alterations were observed in cells after exposure to ULS;

- Cell shrinkage

- Membrane blebbing

- Chromatin condensation

- Nuclear fragmentation

- Apoptotic body formation

In US patent no. 5,984,882 a methodology for the treatment of cancer by introducing or stimulating apoptosis with ultrasonic energy is provided. The frequency level of ultrasound energy described in this document is in the range of 1 kHz to 3 kHz, typically 2 kHz in vitro, in vivo and in human pre cancerous, cancerous and target cell studies, and 15 kHz to 250 kHz, typically 45 kHz in in situ cancer studies. Furthermore, tumour cell are suggested to be irradiated with a power of about 20 W+/- 0.2 W in vivo, and of about 12.0 W+/- 0.9 W in humans.

The total dosage of ultrasonic energy supplied is stated to be set above at least 22.5 W/s (J) and the cavitation threshold of blood.

### 4.2 Acoustic energy in combination with conventional treatment

The application of conventional cancer treatments, and in particular the application of chemotherapy, have been well documented in the literature.

US patent applications no. 20010007666 and 20010002251 provide methodologies for the combination of various substances with ultrasonic sound for selective cell destruction.

Empirical evidence supporting the hypothesis of selective cell destruction by the combination of chemicals and acoustics is provided in the literature. Wörle, Steinbach, Hofstädter (1994) studied the combined effects of high-energy shock waves and cytostatic drugs or cytokines on human bladder cancer cells. From these studies they concluded that transient shock wave-induced permeabilisation of the cell membrane not only enhanced drug efficiency, but also damaged cell organelles and caused alternations in cellular metabolism.

Maruyama et. al. (1999) studied the application of high energy shock waves to cancer treatment in combination with cisplatin and ATX-70 both in vitro and in vivo. They concluded that high-energy shock waves activated or enhanced ATX-70, and that the anti-tumour effect of high-energy shock waves and ATX-70 was caused by generation of active oxygen species. They also concluded that high-energy shock waves could be combined with any other cancer treatment.

Kato et. al. (2000) investigated the mechanism of anti-tumour effect by the combination of bleomycin and shock waves. In this study they evaluated the synergistic effects on cancer cell proliferation and apoptosis in solid tumours. A human colon cell line, SW 480, was implanted onto the back of nude mice. Two thousand shock waves were administered to the tumour immediately followed by an intravenous injection of bleomycin at a dose of one-tenth of the $LD_{50}$. They concluded that shock waves may enhance chemotherapeutic effects by increasing apoptosis and decreasing cell proliferation in the tumour tissue.

## 4.3 By inducing oscillations of natural (mechanical) frequency

Any body or systems of bodies, both physical and biological, has or can oscillate at various natural frequencies. Based on the significant differences in internal and external structure, there are qualified reasons to believe that the mechanical resonance frequencies to normal cells and the equivalent for cancer cells are quite different.

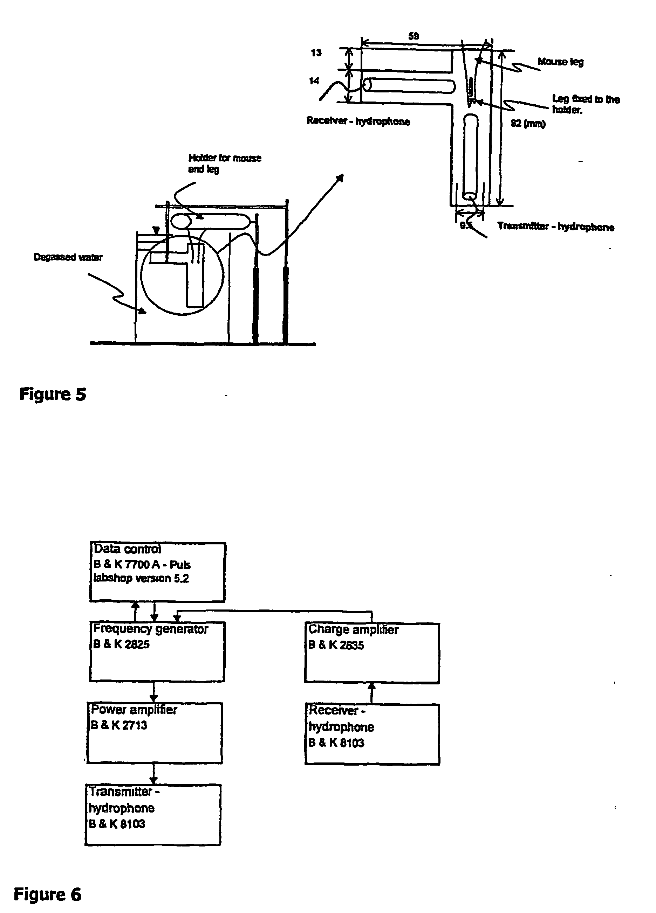

Empirically determination of natural or resonance frequencies can be done by studying the amplitude rate (reference to paragraph 4.3.1) by the use of standard commercially available laser interferometers, or by the use of the described set-ups or apparatuses by measuring selectively energy absorption rates, with reference to **figure 6.**

For a treatment of mechanical properties of cells, one may refer to Bereither-Hahn (1987).

## 4.3.1 A biomechanical model of a cell exposed to an external mechanical force

A first step in the process of developing a procedure and system for selective cell and virus destruction, based on the application of (mechanical) natural frequencies, is the development of a biomechanical cell model which utilises the differences in mechanical resonance frequency between cancer cells and normal cells.

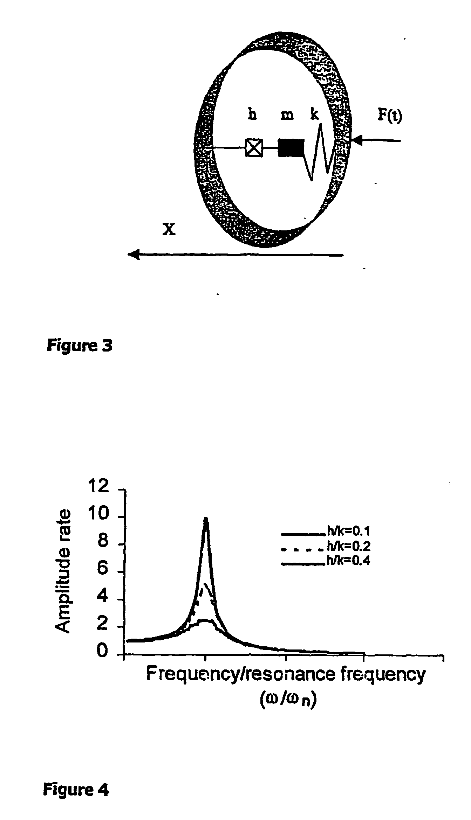

Based on an external energy source (harmonic acoustic frequency generator) this causes the cell to be excited by a subsequent external force, F(t). See figure 3 for a schematic presentation. The cell system is modelled as a body with mass, m, in combination with a spring with a spring stiffness, k, and with hysteresis damping characteristics, represented by a hysteresis damping constant, h. Hysteresis damping is a form of internal damping which includes a combination of both viscous damping, where the damping is proportional with velocity, and coulomb damping, which is friction damping where the damping force is constant. Hysteresis damping is based on internal friction or hysteresis which occur when a well defined body is deformed. When a body is subjected to repeated elastic strain, thermal effects will occur. At higher frequencies the available time for heat transfer reduces and the damping effect is reduced.

For a classical treatment and discussion of hysteresis damping see Drew (1974).

The equation for the system represented by figure 3 may be represented by;

$$m\,dx^2/d^2t + (h/\omega)dx/dt + kx = F(t) \tag{1}$$

where $\omega$ is the angular frequency of the external force in radians per second ($\omega = 2\pi f$).

By only considering the steady state response of a sinusoidal external force;

$$F(t) = F_1 \sin \omega t \text{ or } F(t) = F_1 e^{i\omega t} \tag{2}$$

The movement or response will be harmonic;

$$x = X \sin (\omega t - \phi) \tag{3}$$

where

$$X = F_1/m[(\omega_n^2 - \omega^2)^2 + (h/m)^2]^{1/2} \tag{4}$$

which represents the maximum response and where
$\omega_n$ = the natural or resonance frequency.

By reorganising (4) one may define the concept amplitude rate in the following way;

$$\text{Amplitude rate} = X/(F_1/k) = 1/[(1-\omega^2/\omega_n^2)^2 + (h/k)^2]^{1/2} \tag{5}$$

and the subsequent phase angle, $\phi$;

$$\tan \phi = h/m(\omega_n^2 - \omega^2) = h/k(1 - \omega^2/\omega_n^2) \tag{6}$$

The force to the foundation, which means the cell membrane, will be;

$$kx = kX \sin(\omega t - \phi) \tag{7}$$

from the damping characteristics or component
and

$$(h/\omega)dx/dt = hX \cos(\omega t - \phi) \tag{8}$$

from the hysteresis properties, respectively.

The system is assumed to be exposed by a pressure field. It is assumed that a transmitter generates oscillating pulses which again induces a pressure field p(r,t).

If one by the expression, p(r,t), assumes the real part of the expression, it can be shown that the pressure within a harmonic spherical diverging sound wave may be represented by the expression;

$$p(r,t) = (A/r)e^{i\omega(t- r/v)} \tag{9}$$

where

A = complex constant

r = distance to the source

$\omega$ = angular frequency to the sound wave (pulses)

v = velocity of sound within the matter (cytoplasm)

The particle velocity, u(r,t), can be shown to be represented by the real part of the expression;

$$u(r,t) = (A/i\omega\rho_o)\{i\omega/cr + 1/r^2\}e^{i\omega(t-t/v)} \tag{10}$$

where
$\rho_o$ = density of the matter (in the absence of an acoustic source)
Equivalently for a plane wave;

$$p = (A/r)e^{i\omega(t-x/v)} \tag{11}$$

and

$$u = p/\rho_o v \qquad (12)$$

The external force to the cell, $F_1$, will subsequently be represented by p(r,t) multiplied by a representative cross sectional area of the cell.

Related to the intensity of the acoustic transmitter, where I = Efficiency (watt)/area = $P/2\pi r^2$ and represents acoustic energy per unit of area of space, the following expression may be derived;

For a plane source against a flat surface;

$$I = p^2/2\rho v \qquad (13)$$

For pulsating spherical source;

$$I = P/2\pi r^2 \qquad (14)$$

where

$$P = 2\pi\rho_o\varepsilon^2 a^4\omega^4/[v(1 + (\omega a/v)^2] \qquad (15)$$

a = radius of the oscillator

$\varepsilon$ = amplitude of oscillator

Figure 4 represents a presentation of the amplitude rate (equation 5) as a function of $\omega/\omega_n$ and the relation between the hysteresis damping (h) and the spring stiffness (k).

At $\omega = \omega_n$ maximum amplitude rate is achieved.

$(X/F_1/k)$ approaches infinity when h/k -> 0, equivalently $(X/F_1/k)$ reduces with increasing h/k - relationship.

Based on the above stated cell model, the angle of approach, related to the development of an apparatus and algorithm for selective cell and virus destruction, will be the following;

Empirically determine optimal resonance frequency(ies) $\omega_n$ for different cancer and/or tissue types, if necessary also as a function of time (stage). Means of obtaining resonance frequencies may be by measurement of actual amplitude rates or selective frequency absorption rates within the relevant tissue. The optimal resonance frequency for various cancers or tissue types, is defined as the or those natural frequencies that are (furthest) apart from equivalent frequencies for normal tissue or organs, provided that they represent sufficiently pressure extortion to obtain selective cell destruction, and at the same time may induce apoptosis/necrosis in cancer cells.

The solution to the above stated problem, based on the relationship; $F_1 = f(p) = f(I,r)$, provided m = $\omega_n$, empirically determine $F_1$ in such a way that one obtains cell necrosis in combinations of membrane rupture, nucleus membrane and/or organelles are destructed by shear stress, $\tau = f(F_1)$, $\tau > \tau_{crit}$ and/or local (internal) thermal (dissipative) effects (within a cell). Subjected to the qualified assumption that $\omega_{n\ cancer\ tissue}$ (n represents various cancer types) is significantly different from $\omega_{n\ normal\ tissue}$ at intensities less than, but not necessarily, cavitational levels and/or combined with a frequency band which causes apoptosis/necrosis in cancer cells, provided an accumulated energy level, W, W > $W_{apoptosis}$, in parallel or sequentially by applying an additional frequency which induces apoptosis, $\omega_{apoptosis}$, provided W > $W_{apoptosis}$, and/or in combination with traditional therapies, like chemotherapy, in combination with acoustic energy.

### 4.3.2 Some empirical evidence of selective natural frequencies

Experiments have been conducted with Balb/c nude mice with WiDr tumours transplanted to one of the back legs.

With the use of an experimental set up as described in figure 5, mice were placed in a holder with one leg inserted into and from the top of a T-arrangement. The T-structure had a hydrophone in transmitter mode placed vertically underneath and an equivalent hydrophone in receiver mode was placed horizontally. The mouse leg with the T-arrangement and the transducers were submerged into a beaker filled with degassed water. The set up was hooked up with both hardware (frequency generator, power amplifier etc) and software from Brüel & Kjær, in accordance with block diagrams as described in figure 6. The setting was; frequency range 1 Hz - 25 kHz, sweep time 1 kHz/s, 1 Vmrs, 1 A and 30 + 5 dB.

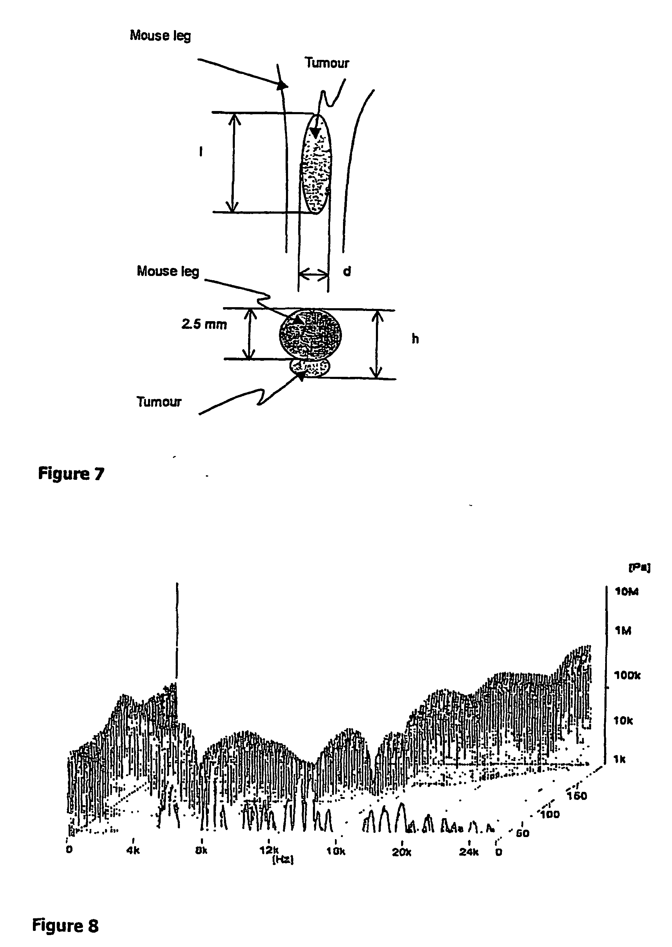

The tumour specifications for the specific mouse represented by diagrams 8 and 9, are; length 7.4 mm, diameter 4.7 mm, and height 6.0 mm including a mouse leg thickness of 2.5 mm. Both back legs had a length of 18.0 mm. For a schematic presentation of the leg with tumour see figure 7.

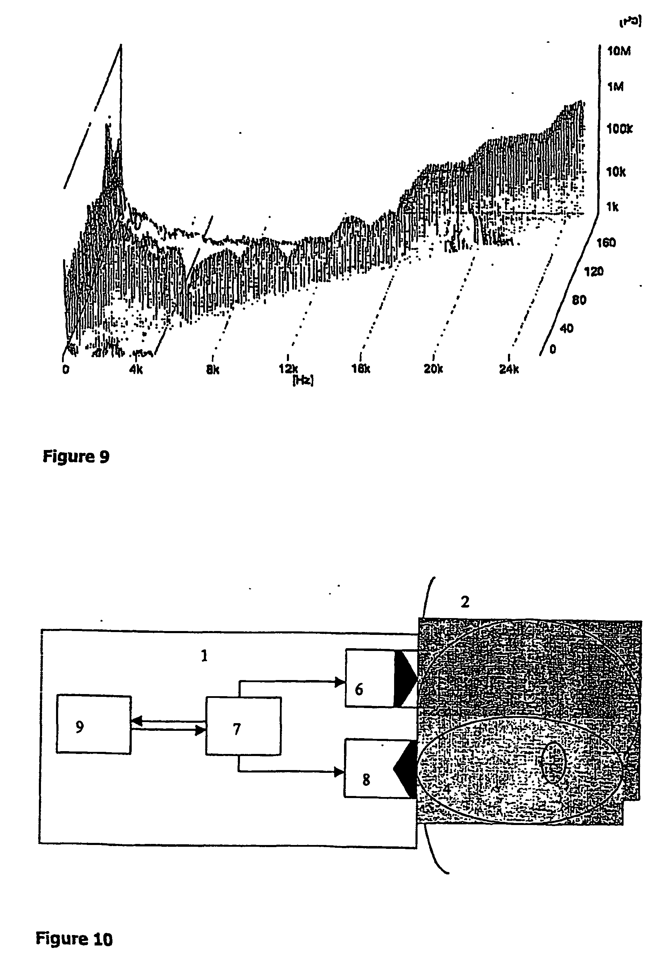

Figure 8 and 9 represents absorption rates (in Pascal) as a function of frequency for a healthy back leg, and a back leg with tumour for a Balb/c nude mouse with the above stated characteristics.

Table 1

| Frequencies where the system with healthy leg and leg with tumour showed significant energy absorption. | | | |
|---|---|---|---|
| | | *Frequency (kHz)* | |
| *Healthy leg* | *3.5* | *8.5* | *14* |
| *Leg with tumour* | *5.5* | *7.5* | *10* |

As seen from table 1, which is based on figures 8 and 9, there are significant differences at which frequencies the various systems (healthy leg vs. tumour leg) provides energy absorption.

## 5. An apparatus and a procedure for guidance and control with respect to selective cell and virus destruction

### 5.1 General

The biomechanical model which has been developed assumes an externally induced harmonic oscillating pulse within sonic or ultrasonic frequencies.

The use of ultrasonic energy have previously been used in various medical applications, to interfere with tissue or other materials in several applications. US patents no. 4,989,588 and 4,867,141 describe methods of applying ultrasound to crush kidney stones. US patents no. 5,694,936 and 5,413,550 describe apparatuses for the increase of tissue temperature for hyper-thermal treatment. US patents no. 5,899,857, 5,827,204 and 5,209,221 describe different forms of acoustical surgery by the use of cavitation and the destruction of tissue. An equivalent device is provided by US patent no. 6,113,558 which includes a cavitation generating tip at the distal end of an elongated transmission member. The apparatus and method could assist in the treatment of medical conditions such as cancer. US patent no. 5,165,412 describes an apparatus that provides shock waves into the body to destroy tissue within a focus point. US patents no. 5,725,482 and 5,664,570 describe methodologies to generate standing ultrasonic waves from several transmitters focused into a well defined point within the body (focus point).

The major problem with all these apparatuses/methods is that, equivalently as discussed under paragraph 2, to differentiate between normal and cancer tissue. Also, in cancer applications, the above mentioned procedures will require a well defined tumour/location, because the procedures target an well defined volume (focus point), independent of tissue or tissue type.

Due to general conductive thermal effects (from treatment) substantial collateral damage may be incurred on healthy tissue. In addition, normal fluid flow within the body may cause substantial heat transfer (cooling) such that thermal treatment may be rather cumbersome in practical applications.

### 5.2 Apparatus

A very central concept within acoustics is acoustic impedance ("acoustic ohm"). It represents the complex relationship between sound pressure on a moveable surface and the volume-velocity of the surface (velocity x area). When sound waves penetrate matter, this causes a pressure reduction or attenuation due to energy absorption, reflection and diffraction.

Empirically attenuation has been stated as a function of frequency.

$$\text{Coefficient of attenuation} = \alpha_p = f(\omega) \tag{16}$$

Reflection and diffraction occur at the boundary layer(s) between areas with different acoustic impedance. In medical ultrasound in general and also related to the provided apparatus, the basic principle is that differences in acoustic impedance occur between opposite sides of different boundaries (organs) and that these boundaries provide significant reflections.

For a plane wave the intensity (I) as a function of tissue depth or thickness (r) (minus the diffraction component), can be modelled as;

$$I(r) = I_0 \, e^{-\mu(f)r} \qquad (17)$$

where

$I_0$ = output intensity
$\mu(f)$ = Intensity-absorption coefficient, which is a function of frequency, f ($\omega = 2\pi f$)

$$\mu(f) = A(f/f_1)^b = A(\omega/\omega_1)^b \qquad (18)$$

where

A and b are dependent of type of tissue.

The absorption coefficient per unit of wave length ($\lambda$) may be represented by the following expression, where v = velocity of sound in the tissue and $\lambda$ = v/f;

$$\mu\lambda = Avf^{(b-1)}/f_1^b \qquad (19)$$

For further discussions, reference may made to Hedrick et. al. (1995) and Robb (1995).
With reference to figure 10, there is provided an apparatus (1) for selective cell or virus destruction within a living organism (2) like a human being, an animal or a plant The apparatus comprises

- an acoustic transmitter (6) which is arranged to transmit an acoustic signal into a first portion (3) of the organism, and
- a control unit (7) designed to control characteristics associated with the acoustic signal, including to set the frequency for at least one component of the signal to a determined frequency value which causes specific cells (5) within the first cellular portion (3) to be damaged or destroyed under the influence of the signal.

The control unit (7) is arranged to control the power of the acoustic signal during a period of exposure in such a way that the total energy of the signal during the period of exposure does not exceed a critically accumulated energy level.
Further, the control unit (7) is arranged to obtain a resonance frequency value associated with properties for the specific cells (5) from the computer device (9), and the control unit (7) is designed to use said resonance frequency value as the determined frequency value.
Further, the guidance and control unit (7) is arranged to obtain an apoptotic or necrototic frequency value associated with properties for the specific cells (5) from the computer device (9), and the control unit (7) is arranged to use said apoptotic or necrototic frequency value as the determined frequency value.
Further, the control unit (7) is arranged in such a way that, from the computer device (9), a frequency value is obtainable in combination with a chemotherapeutic drug associated with properties for the specific cells (5), and the control unit (7) is arranged to use said frequency in combination with the stated chemotherapeutic drug as the determined frequency and chemotherapeutic drug.
Further the control unit (7) is arranged to obtain a critical power level associated with properties for the specific cells (5) from the computing device (9), and the control unit (7) is arranged to control the power of the acoustic signal in such a way that it will not exceed the critical power level.

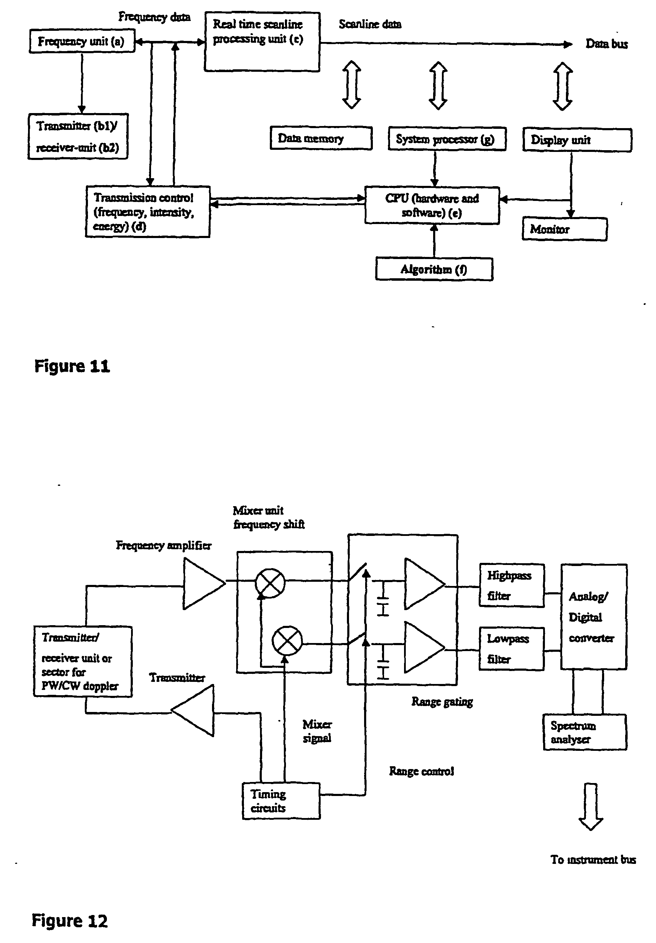

Figure 11 provides a block diagram of an apparatus, based on the overall scheme provided by figure 10, which may generate both tissue monitoring and provide treatment for cancer.
The overall objective of the system is treatment, secondary monitoring for diagnostic purposes. The apparatus consists of frequency generator(s), transmitter(s)/ receiver(s), scanline processors, central processing unit (CPU), system processors, scanconverter display unit etc.
The link between figure 10, which represent overall concepts, and figure 11, which outlines a block diagram of an actual system (apparatus), are represented by the comparative elements outlined in table 2 below:

Table 2

| Comparison of conceptual elements of figure 10 with actual system components of figure 11. | |
|---|---|
| *Figure 10* | *Figure 11* |
| Acoustic transmitter (6) | Frequency unit (a), transmitter (b1) |
| Guidance and control unit (7) | Transmission control (frequency, intensity, energy (d), central processing unit (e) |
| Absorption measuring device (8) | Receiver unit (b2), real time scanline unit (c), central processing unit (e) |
| Computer arrangement (9) | Central processing unit (e), algorithm (f), system processor (g) |

The scanline processors are central in the sense that they analyse reflected signals from each scanline, where the primary objective, in an therapeutic context, is measurement and calculations of intensities and energy levels along scanlines, in such a way that the system endogenously calculate intensities and energy levels along vectors, or at the end point of vectors, where the vectors define the location of tumours. These calculated values represent endogenous parameters within the CPU and transmission control units, which control the transmitter function in accordance with reference values for intensities and energy levels.

The system with scanline processor(s) also treat and produce data which are not necessary from a therapeutic point of view, but which are up to date with respect to tissue imaging. The scanline processor processes the scanline signals with respect to (2D) dimensional data (and image), time motion data (and display) and colour flow image. The latter is achieved by the combination of Doppler signals and (2D) echo signals.

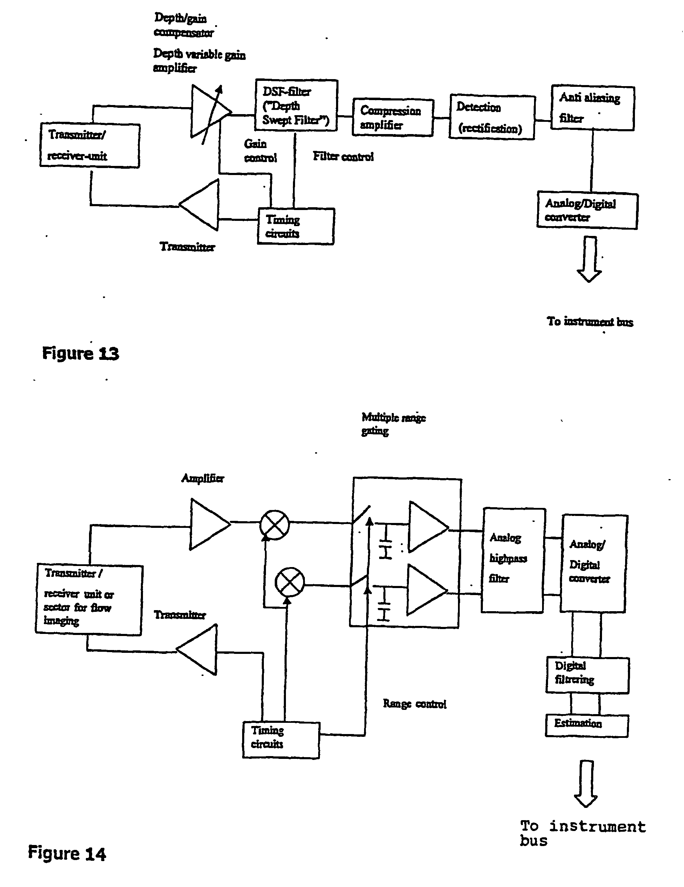

Figures 12, 13 and 14 represent block diagrams of system configurations with respect to scanline processing for tissue imaging, flow imaging and PW/CW Doppler measurements, respectively. In figure 12 scanline processing of reflected signals as a function of tissue depth is shown, where circuits, type of amplifiers, different filters etc are placed. Figure 13 represents a block diagram for scanline processing of PW and CW Doppler signals, where the Doppler signals are the modulated frequency signal which frequency is the Doppler frequency. Within the diagram is the arrangement of amplifiers, mixer units, filters, transformers etc. Figure 14 provides a block diagram for scanline processing for colour flow imaging. The various components (filters, circuits etc) of the system are shown.

Related to dynamic tissue imaging (in colours), the relevant signal parameters are extracted and transmitted via a standard computer bus to a scanconverter display unit and monitor. Data may also be transferred to a data memory unit to freeze pictures or transferred to an external CPU for further processing.

For a general reference and comparison to the design and structure of ultrasound apparatuses, one may refer to e.g. Angelsen (1996).

Based on (external) mechanical influence, and energy intensities at the location of the tumour is above a critical level, with or without cavitation, one may obtain combinations of thermal effects and shear stress within cancer cells in such a way that the cell or nucleus membranes rupture and/or in combination with damage to organelles. With apoptotic/necrotic processes in combinations with or instead of resonance effects, with or without additional combinations of chemotherapeutic treatment in combination with acoustic shocks, one may attack cancer cells selectively from various angels (simultaneously), and in such way maximise the probability of selective cancer cell destruction.

Additionally, the procedure will represent treatment of a larger tissue area/body part (none focused treatment) with a minimum of risk for damage to adjacent tissue or organs.

The determination of natural frequencies by the use of amplitude rates to cells, both cancer cells and normal cells, or in combination with apoptotic/necrotic frequencies and subsequent accumulated energy levels, may be performed by either exposing them with acoustic transmission from a (standard) piezoelectric crystal and/or other (standard) transducers, in combination with a pulse generator, where the movements (amplitude rates) to the various cells can be registered by a laser interferometer with sufficient sensitivity, or the energy absorption rates are measured with the use of several transducers, with reference to the experimental set up of figure 6.

Intensities and accumulated energy levels for the destruction of various cancer/tissue types, with the possibility of function of stage (time), are established experimentally, together with thresholds to prohibit (reduce) damage to healthy tissue. Equivalent empirical analyses are conducted to establish separate apoptotic/necrotic frequencies and subsequent accumulated energy levels.

Based on the above stated empirical procedures the following are established:

A library of empirical data for (optimum) mechanical resonance, $\omega_{cn}$ (t), where n represents various (optimum) resonance frequencies, for different cancer/tissue types, c, with the additional possibility as a function of stage (time, t).

A library of empirical data for apoptotic/necrotic frequencies, $\omega_{c\,apoptosis\,m}$ (t), where m represent various values for apoptosis frequencies, for different cancer/tissue types, c, with the additional possibility as a function of stage (time, t).

A library of empirical data for chemotherapeutic drug, d, with corresponding (shock) frequencies, $\omega_{dmc}$ (t),

where dm represents various frequencies, for different cancer/tissue types, c, with the additional possibility as a function of stage (time, t).

A library of empirical data of critical intensity levels, Ic $_{critical\ \mu}$ (t), where Ic $_{critical\ \mu}$ (t) represents critical intensity levels with respect to selective cell destruction for different cancer/tissue types (including normal tissue), c, with the additional possibility as a function of stage (time, t) with respect to $\mu$. $\mu$ represent optimum resonance frequency, at resonance frequency isolated, at apoptosis/necrosis frequency isolated or the application of chemotherapeutic drug, d, with corresponding (shock) frequency isolated.

A library of empirical data for critical energy levels, Wc $_{critical\ \mu}$ = Ic $_{critical\ \mu}$ T critical (t), where Wc $_{critical\ \mu}$ represent critically accumulated energy levels (per unit of area) for different cancer/tissue types (including normal tissue), c, with the additional possibility as a function of stage (time, t) with respect to $\mu$.

The vector(s) to the tumour(s)/metastasis(es), combined with scanline data, will provide initial energy efficiencies and intensities to the various transducers and calculate intensities and accumulated energy levels at the vector (s) end point(s) (tumours). These procedures represent endogenous processes.

The apparatus/system will compare endogenously the calculated relevant intensities and accumulated energy levels with the relevant library values and determine stop or continued transmission of the apparatus, with the possibility of manual bypass.

In a clinical application one will apply the use of gel or fluid to minimise the attenuation between transmission and the object under treatment.

## 5.3 Algorithm

The algorithm for the system for selective cell or virus destruction for human beings, animals or plants, based on the previously stated apparatus, which also includes;

1. A library of empirical data for (optimum) mechanical resonance frequencies, $\omega_{cn}$ (t), where n represents various (optimum) resonance frequencies, for different cancer/tissue types, c, with the additional possibility as a function of stage (time, t).

2. A library of empirical data for apoptotic/necrotic frequencies, $\omega_{c\ apoptosis\ m}$ (t), where m represent various values for apoptosis frequencies, for different cancer/tissue types, c, with the additional possibility as a function of stage (time, t).

3. A library of empirical data for chemotherapeutic drug, d, with corresponding (shock) frequencies, $\omega_{dmc}$ (t), where dm represents various frequencies, for different cancer/tissue types, c, with the additional possibility as a function of stage (time, t).

4. A library of empirical data of critical intensity levels, Ic $_{critical\ \mu}$ (t), where Ic $_{critical\ \mu}$ (t) represents critical intensity levels with respect to selective cell destruction for different cancer/tissue types (including normal tissue), c, with the additional possibility as a function of stage (time, t) with respect to $\mu$. $\mu$ represent optimum resonance frequency, at resonance frequency isolated, at apoptosis/necrosis frequency isolated or chemotherapeutic drug, d, with corresponding (shock) frequency isolated.

5. A library of empirical data for critical energy levels, Wc $_{critical\ \mu}$ = Ic $_{critical\ \mu}$ T critical (t), where Wc $_{critical\ \mu}$ represent critically accumulated energy levels (per unit of area) for different cancer/tissue types (including normal tissue), c, with the additional possibility as a function of stage (time, t) with respect to $\mu$.
Which in addition utilises the following algorithm;

6.1 The application of the previously defined apparatus, with the possibility of standard diagnostic procedures, like combinations of x-ray/CT, ultrasound, MR, biopsy etc, a specific cancer is diagnosed, c (t), and the location of tumour(s) and or metastases are located, which defines the vector(s) to the tumour(s).

6.2 In combination with one or more of the following treatment options;
Use of optimum frequencies, apoptosis/necrosis and resonance frequencies coincide

6.2.1 Subjected that $\omega_{cn}$ (t) = $\omega_{c\ apoptosis}$ (t), if not go to 6.2.2.
Given vector(s) to c.

6.2.1.1 Pursue transmission defined by frequencies in accordance with paragraph 1.

6.2.1.2 Where initial intensities and total energy is maximised, subjected to thresholds provided by paragraphs 4 and 5, $W > W_{apoptosis}$.

Use of resonance frequency isolated - apoptosis/necrosis frequencies are different

6.2.2 Subjected that $\omega_{cn}$ (t) $\lozenge$ $\omega_{c\ apoptosis}$ (t)
Given vector(s) to c.

6.2.2.1 Pursue transmission defined by frequencies in accordance with paragraph 1.

6.2.2.2 Where initial intensities and total energy is maximised, subjected to thresholds provided by paragraphs 4 and 5.

Use of apoptosis/necrosis frequency isolated - resonance frequencies are different

6.2.3 Subjected that $\omega_{c\ apoptosis}$ (t) $\lozenge$ $\omega_{cn}$ (t)
Given vector(s) to c.

6.2.3.1 Pursue transmission defined by frequencies in accordance with paragraph 2, $W>W_{apoptosis}$.

6.2.3.2 Where initial intensities and total energy is maximised, subjected to thresholds provided by paragraphs 4 and 5.
Use of chemotherapeutic drugs and acoustic shock isolated
Given vector(s) to c.

6.2.4.1 Pursue transmission defined by frequencies in accordance with paragraph 3.

6.2.4.2 Where initial intensities and total energy is maximised, subjected to thresholds provided by paragraphs 4 and 5.
Combined use of resonance frequency, apoptosis/necrosis frequency and chemotherapeutic drugs in combination with acoustics
Given vector(s) to c.

6.2.5.1 Pursue sequential and/or parallel transmissions defined by frequencies and d in accordance with paragraphs 1, 2 and 3, $W>W_{apoptosis}$.

6.2.5.2 Where initial intensities and total energy is maximised, subjected to thresholds provided by paragraphs 4 and 5.

6.2.6 In combination with paragraphs 6.2.1 to 6.2.5 with the additional application with other conventional treatment options.

6.2.7 In combination with paragraphs 6.2.1 to 6.2.6 where tissue or organs have been exposed to transducers with in situ location via a catheter, probe or any other similar instrument.

6.2.8 In combination with paragraphs 6.2.1 to 6.2.7 where data provided by paragraphs 1 through 5 are substituted by specific data based on an biopsy from a specific human being (or animal or plant) with respect to cancer.

6.2.9 In combination with paragraphs 6.2.1 to 6.2.8 with the adjustments to other types of cells or viruses.

The present invention is not limited to the described apparatus it is intended to include all modifications within the scope of protection provided by the appended claims. Various arrangements for treatment of body tissue or fluid, for example blood, outside the body in a special container, different arrangements for in situ treatment, through tubes or veins/arteries or by natural orifices of the body, various sizes and geometric designs of transducers, automatic control and guidance of transducers, the combination of the apparatus with MR or any other scanning device for real time necrosis detection for real time feedback to the guidance and control unit, the placement of transducers on moveable fixtures, arrangements for servicing and the handling of patients by moveable and/or automatic benches, built in or partly or totally integrated systems or solutions, as far as they are covered by the scope of protection provided by the claims, are all obvious variations to be derived by a skilled person in the art, subjected that this description of the stated invention is provided. All drawings and figures are to be interpreted illustratively and not in a limiting context.

## 6. References

### 6.1 Literature

Alberts, A. et al. (1995); "Molecular Biology of the Cell", Garland Publishing Inc., USA.

Angelsen, B.A. J. (1996); "Waves, signals and signal processing in medical ultrasonics", Norwegian University of Science and Technology, Trondheim, Norway.

Ashush, H., Rozenszajn, L. A., et. al. (2000); "Apoptosis induction of human myeloid leukemic cells by ultrasound exposure", Cancer Res. Feb 15;60(4).

Bereiter-Hahn, J. (1987); "Mechanical principles of architecture of eukaryotic cells", "Cytomechanics", Bereiter-Hahn, J., et. al. (ed), Springer-Verlag, Tyskland.

Drew, J. H. (1974); "Stability of certain periodic solutions of a forced system with hysteresis", International Journal of Non-Linear Mechanics, 9.

Fleming, I. D. (1997); "American Joint Committee on Cancer - Cancer Staging Manual", Lippincott Williams & Wilkins Publishers, USA.

Hedrick, W. R., et. al. (1995); "Ultrasound physics and instrumentation", Mosby, USA.

JNCI, (2001); Database, Journal of The National Cancer Institute, USA.

Kato, M., Ioritani, N., et. Al (2000); "Mechanism of anti-tumour effect of combination of bleomycin and shock waves", Jpn J Cancer Res Oct;91(10).

Kumar, S. (ed). (1999); "Apoptosis, biology and mechanisms", Springer- Verlag, Germany.

Maruyama, M., Asano, T., et al. (1999); "Application of high energy shock waves to cancer treatment in combination with cisplatin and ATX-70", Anticancer Res May-June;19(3A).

NCI, (2001); Database, National Cancer Institute/CancerNet, USA .

NIH, (2001); "Computational Molecular Biology - Databases", National Institute of Health, USA.

Robb, R. A. (1995); "Three-dimensional biomedical imaging: principles and practice", VCH, USA.

Wörle, K., Steinbach, P., Hofstadter, F. (1994); "The combined effects of high-energy shock waves and cystostatic drugs or cytokines on human bladder cancer cells", Cancer Jan;69(1).

### 6.2 Patents

DE 44 14 239, (1994), Vorrichtung zur Behandlung von krankhaften Zellen im lebenden Körper.

US patent no. 6,113,558, (2000), Pulsed mode lysis method.

US Patent no. 5,984,882, (1999), Method for prevention and treatment of cancer and other proliferative diseases with ultrasonic energy.

US Patent no. 5,908,441, (1999), Resonant frequency therapy device.

US Patent no. 5,899,857, (1999), Medical treatment method with scanner input.

US Patent no. 5,827,204, (1998), Medical noninvasive operations using focused modulated high power ultrasound.

US Patent no. 5,725,482, (1998), Method for applying high-intensity ultrasonic waves to a target volume within a human or animal body.

US Patent no. 5,664,570, (1997), Apparatus for applying high-intensity ultrasonic waves to a target volume within a human or animal body.

US Patent no. 5,413,550, (1995), Ultrasound therapy system with automatic dose control.

US Patent no. 5,694,936, (1997), Ultrasonic apparatus for thermotherapy with variable frequency for suppressing cavitation.

US Patent no. 5,209,221, (1993), Ultrasonic treatment of pathological tissue.

US Patent no. 5,165,412, (1992), Shock wave medical treatment apparatus with exchangeable imaging ultrasonic wave probe.

US Patent no. 4,989,588, (1991), Medical treatment device utilizing ultrasonic wave.

US Patent no. 4,867,141, (1989), Medical treatment device utilizing ultrasonic wave.

US Patent no. 4,622,952, (1986), Cancer treatment method.

US Patent no. 4,315,514, (1982), Method and apparatus for selective cell destruction.

**6.3 Patent applications**

US patent application no. 20010007666, (2001), Enhanced transport using membrane disruptive agents.

US patent application no. 20010002251, (2001), Intracellular sensitizers for sonodynamic therapy.

**Claims**

1. Apparatus (1) for selective cell destruction within a living organism (2) comprising

   - an acoustic transmitter (6) which is arranged to transmit an acoustic signal into a first portion (3) of the organism, and

   - a control unit (7) designed to control characteristics associated with the acoustic signal, including to set the frequency for at least one component of the signal to a determined frequency value which causes specific, cancerous cells (5) within the first cellular portion (3) to be damaged or destroyed under the influence of the signal,

   - the apparatus further comprising an absorption measuring device (8) which provides acoustic absorption data from a second portion of the organism, said second cellular portion (4) including the specific cells (5),

   - the control unit (7) being arranged to obtain a resonance frequency value or an apoptotic frequency value associated with properties for the specific cells (5) from a computer device (9),

   - the control unit (7) being further arranged to use said resonance frequency value or apoptotic frequency value as the determined frequency value, and

- the control unit (7) being further arranged to control the power of the signal, based on the absorption data provided by the absorption measuring device (8),

    **characterised in**

- **that** the control unit (7) is further arranged to obtain from the computer device (9) a critical accumulated energy level associated with properties of the specific cells (5), and
- **that** the control unit (7) is further arranged to control the power of the acoustic signal during a period of exposure in such a way that the total energy of the signal during the period of exposure does not exceed the critical accumulated energy level.

2. Apparatus in accordance with claim 1,
   **characterised in**
   **that** the control unit (7) is arranged to obtain a critical power level associated with properties for the specific cells (5) from the computer device (9), and
   **that** the control unit (7) is arranged to control the power of the acoustic signal in such a way that it will not exceed the critical power level.

3. **Apparatus in accordance with daim 1 or 2,**
   **characterised in**
   **that** the control unit (7) is arranged to obtain from the computer device (9) a frequency value corresponding to empirical data for a chemotherapeutic drug associated with properties for the specific cells (5), and a critical power level associated with properties for the specific cells (5), and
   **that** the control unit (7) is arranged to use said frequency value corresponding to said, empirical data for said chemotherapeutic drug as the determined frequency, and to control the power of the acoustic signal in such a way that it will not exceed the critical power level.

**Patentansprüche**

1. Vorrichtung (1) für eine selektive Zellzerstörung innerhalb eines lebenden Organismus (2) mit

- einem akustischen Transmitter (6), der so angeordnet ist, dass er ein akustisches Signal in einen ersten Bereich (3) des Organismus überträgt, und

- einer Steuereinheit (7), die so ausgebildet ist, dass sie Merkmale steuert, die mit dem akustischen Signal zusammenhängen, einschließlich des Festsetzens der Frequenz für mindestens eine Komponente des Signals auf einen vorbestimmten Frequenzwert, der bewirkt, dass spezifische kanzeröse Zellen innerhalb des ersten Zellbereichs (3) beschädigt oder zerstört werden unter Einfluss des Signals,

wobei die Vorrichtung ferner ein Absorptionsmessgerät (8) umfasst, das akustische Absorptionsdaten vom zweiten Bereich des Organismus aufnimmt, wobei der zweite Zellbereich (4) die spezifischen Zellen (5) enthält,
wobei die Steuereinheit (7) so angeordnet ist, dass sie einen resonanten Frequenzwert oder einen apoptotischen Frequenzwert als den festgestellten Frequenzwert verwendet, und
wobei die Steuereinheit (7) weiterhin so angeordnet ist, dass sie die Energie des Signals steuert, basierend auf den Absorptionsdaten, die durch das Absorptionsmessgerät (8) zur Verfügung gestellt werden,
**dadurch gekennzeichnet,**
**dass** die Kontrolleinheit (7) ferner so angeordnet ist, dass sie von der Computereinnchtung (9) eine kritische Schwelle der angesammelten Energie erhält, die mit Eigenschaften der spezifischen Zellen (5) im Zusammenhang stehen, und
**dass** die Steuereinheit (7) weiterhin so angeordnet ist, dass sie die Energie des akustischen Signals während einer Bestrahlungsdauer derart steuert, dass die Gesamtenergie des Signals während der Bestrahlungsdauer nicht die kritische Schwelle der angesammelten Energie übersteigt.

2. Vorrichtung nach Anspruch 1, **dadurch gekennzeichnet, dass** die Steuereinheit (7) so angeordnet ist, dass sie eine kritische Energieschwelle, die mit Eigenschaften der speziellen Zellen (5) zusammenhängt, von der Computereinrichtung (9) erhält, und dass die Steuereinheit (7) so angeordnet ist, dass sie die Energie des akustischen Signals derart steuert, dass sie die kritische Energieschwelle nicht übersteigt.

3. Vorrichtung nach Anspruch 1 oder 2, **dadurch gekennzeichnet, dass** die Steuereinheit (7) so ausgebildet ist, dass sie von der Computereinrichtung (9) einen Frequenzwert, der empirischen Daten für ein chemotherapeutisches Mittel, das mit den Eigenschaften der spezifischen Zellen (5) zusammenhängt, und eine kritische Energieschwelle erhält, die im Zusammenhang mit Eigenschaften der spezifischen Zellen (5) steht, wobei die Steuereinheit (7) so ausgebildet ist, dass sie diesen Frequenzwert, der den empirischen Daten für das chemotherapeutische Mittel entspricht, als die festgestellte Frequenz verwendet, und sie die Energie des akustischen Signals derart steuert, dass sie nicht die kritische Energieschwelle übersteigt.

**Revendications**

1. Appareil (1) pour la destruction sélective de cellules dans un organisme vivant (2), comprenant :

   - un émetteur acoustique (6) conçu pour émettre un signal acoustique dans une première partie (3) de l'organisme ; et
   - une unité de commande (7) conçue pour commander les caractéristiques associées au signal acoustique, y compris pour régler la fréquence d'au moins une composante du signal à une valeur de fréquence prédéterminée qui endommage ou détruit des cellules cancéreuses spécifiques (5) dans la première partie cellulaire (3) sous l'influence du signal ;

   - ledit appareil comprenant en outre un dispositif de mesure d'absorption (8) qui fournit des données d'absorption acoustique depuis une seconde partie de l'organisme, ladite seconde partie cellulaire (4) comprenant les cellules spécifiques (5) ;
   - l'unité de commande (7) étant conçue de manière à obtenir d'un dispositif informatique (9) une valeur de fréquence de résonance ou une valeur de fréquence d'apoptose associée au propriétés des cellules spécifiques (5) ;
   - l'unité de commande (7) étant en outre conçue pour utiliser ladite valeur de fréquence de résonance ou ladite valeur de fréquence d'apoptose comme la valeur de fréquence déterminée ; et
   - l'unité de commande (7) étant encore conçue de manière à commander la puissance du signal en fonction des données d'absorption fournies par le dispositif de mesure d'absorption (8) ;

   **caractérisé en ce que** :

   - ladite unité de commande (7) étant en outre conçue de manière à obtenir du dispositif informatique (9), un niveau d'énergie accumulée critique associé aux propriétés des cellules spécifiques (5) ; et **en ce que**
   - l'unité de commande (7) étant en outre conçue de manière à commander la puissance du signal acoustique pendant une période d'exposition, de manière que l'énergie totale du signal pendant la période d'exposition ne dépasse pas le niveau d'énergie accumulée critique.

2. Appareil selon la revendication 1, **caractérisé en ce que** :

   - ladite unité de commande (7) est conçue de manière à obtenir du dispositif informatique (9), un niveau de puissance critique associé aux propriétés des cellules spécifiques (5) ; et **en ce que**
   - l'unité de commande (7) est conçue de manière à commander la puissance du signal acoustique de manière qu'il ne dépasse pas le niveau de puissance critique.

3. Appareil selon la revendication 1 ou 2, **caractérisé en ce que** :

   - l'unité de commande (7) est conçue de manière à obtenir du dispositif informatique (9), une valeur de fréquence correspondant à des données empiriques pour un médicament de chimiothérapie associé aux propriétés des cellules spécifiques (5), ainsi qu'un niveau de puissance critique associé aux propriétés des cellules spécifiques (5) ; et **en ce que**
   - l'unité de commande (7) est conçue de manière à utiliser ladite valeur de fréquence correspondant aux dites données empiriques pour ledit médicament de chimiothérapie comme étant la fréquence déterminée et de manière à commander la puissance du signal acoustique, de sorte qu'il ne dépasse pas le niveau de puissance critique.

Figure 1

Figure 2

**Figure 3**

**Figure 4**

**Figure 5**

**Figure 6**

**Figure 7**

**Figure 8**

**Figure 9**

**Figure 10**

EP 1 313 531 B1

**Figure 11**

**Figure 12**

Depth/gain
compensator

Depth variable gain
amplifier

DSF-filter
("Depth
Swept Filter")

Compression
amplifier

Detection
(rectification)

Anti aliasing
filter

Transmitter/
receiver-unit

Gain
control

Filter control

Analog/Digital
converter

Transmitter

Timing
circuits

To instrument bus

**Figure 13**

Multiple range
gating

Amplifier

Analog
highpass
filter

Analog/
Digital
converter

Transmitter /
receiver unit or
sector for flow
imaging

Transmitter

Range control

Digital
filtering

Timing
circuits

Estimation

To instrument
bus

**Figure 14**

25